# EUROPEAN PATENT SPECIFICATION

(11) **EP 0 339 599 B1**
(45) Date of publication and mention of the grant of the patent: **21.08.1996**
(21) Application number: 89107541.8
(22) Date of filing: 26.04.1989
(51) Int. Cl.: C07D 333/22, C07D 333/24, C07D 333/28, C07C 45/46, C07D 207/32, C07D 409/06, C07D 307/46, C07D 307/54, C07D 307/80, C07D 333/56

(54) **Process for producing acylaromatic compounds**
Verfahren zur Herstellung von acylaromatischen Verbindungen
Procédé de préparation de composés acylaromatiques

(30) Priority: 28.04.1988 JP 107252/88
(43) Date of publication of application: 02.11.1989
(73) Proprietor: SUMITOMO CHEMICAL COMPANY, LIMITED, Chuo-ku Osaka 541 (JP)
(72) Inventor: Minai, Masayoshi, Moriyama-shi (JP); Kondo, Michitada, Higashinada-ku Kobe (JP); Ueda, Yuji, Izumi-shi (JP); Kai, Seiichi, Ikoma-gun Nara-ken (JP); Higashii, Takayuki, Kishiwada-shi (JP)
(74) Representative: Henkel, Feiler, Hänzel & Partner

(56) References cited:
- EP-A- 0 268 820
- CH-A- 547 279
- US-A- 2 492 630
- HOUBEN-WEYL "Methoden der organischen Chemie", vol. VII/2a, 1973, GEORG THIEME VERLAG, Stuttgart, "Ketone, Teil 1", pages 311-331

## Description

The present invention relates to a process for producing acylaromatic compounds represented by the formula 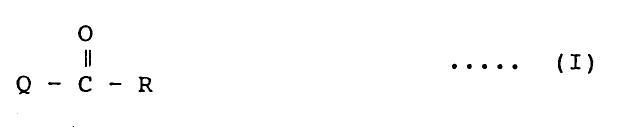 wherein Q is an aromatic compound residue and R is a straight, branched or cyclic aliphatic group, aromatic group or araliphatic group.

The acylaromatic compounds are very important as intermediates of medicines and agricultural chemicals, and are particularly applicable as an intermediate of cholagogue.

The acylaromatic compound can be produced by acylation of an aromatic compound according to Friedel-Crafts Reaction, and the acylation includes the followings;
a) methods of employing an acid chloride and a Lewis acid (aluminum chloride or zinc chloride),
b) methods of employing a carboxylic acid anhydride and a Lewis acid,
c) methods of employing a carboxylic acid and a polyphosphoric acid, and
d) methods of employing a carboxylic acid and trifluoroacetic anhydride (U.S. Patent No. 4,254,043).

However, the method a) necessitates converting a carboxylic acid into the acid chloride with the use of thionyl chloride etc., and further has problems of disposing waste materials containing aluminum or zinc compounds. For the method b) a carboxylic acid anhydride is to be prepared from the corresponding carboxylic acid, and every carboxylic acid is not necessarily converted to its carboxylic acid anhydride which restrict the acylating agent. Methods c) and d) are meritorious industrially due to their allowance for direct usages of carboxylic acids, however, waste water disposals from the large amount of polyphosphoric acid employed and the usage of extremely expensive trifluoroacetic acid anhydride are problems concerned. As mentioned above, the known industrial method for acylation of aromatic compounds for the production of acylaromatic compounds have not necessarily been satisfactory.

Houben-Weyl, Methoden der organischen Chemie, vol. VII/2a, 1973, pages 311-331, discloses acylating processes using carboxylic halides, acid anhydrides etc. and a catalyst. However, these acylating processes show various disadvantages with respect to an industrial application, such as some compounds cannot successfully be acylated with carboxylic halides, and usually acid anhydrides have low reactivities so that only highly reactive aromatic compounds can be used.

EP-A-0268820, published on June 1, 1988, which has to be regarded as a prior art document under Article 54(3) and (4) EPC, discloses a process for producing 2-acylfuran derivatives, represented by the formula 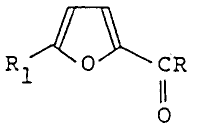 wherein R is alkyl, phenyl, etc. and R₁ is H, alkyl.

The present inventors have ascertained that acylation of an aromatic compound can be achieved with an industrial advantage by the use or a combination of a mixed acid anhydride or a carboxylic acid with an acid anhydride and a boron trifluoride catalyst.
According to the present invention, there is provided a process for producing an acylaromatic compound of the formula (I), which comprises a reaction, in the presence of boron trifluoride or a boron trifluoride complex catalyat, of an aromatic compound, except the compound represented by the formula 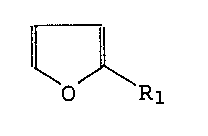 wherein R1 = H or lower alkyl group,
either with
a) a mixed acid anhydride represented by the formula (II) 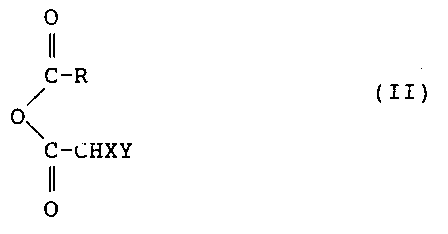 wherein R is a straight, branched or cyclic aliphatic group, aromatic group or araliphatic group, in which the straight, branched or cyclic aliphatic group includes an alkyl group having 1 to 18 carbon atoms, an alkenyl group having 2 to 18 carbon atoms, an alkynyl group having 2 to 18 carbon atoms, a cyclic alkyl group having 3 to 8 carbon atoms, a cyclic alkenyl group having 3 to 8 carbon atoms, a cyclic alkynyl group having 3 to 8 carbon atoms, a cycloalkylalkyl group having 3 to 15 carbon atoms, a cycloalkylalkenyl group having 3 to 15 carbon atoms, a cycloalkylalkynyl group having 3 to 15 carbon atoms, a cycloalkenylalkyl group having 3 to 15 carbon atoms, a cycloalkynylalkyl group having 3 to 15 carbon atoms and a cycloalkenylalkenyl group having 3 to 15 carbon atoms; the araliphatic group includes a thienylalkyl group, a thienylalkenyl group, an phenylalkyl group, a thienylalkynyl group, a furylalkyl group, a naphthylalkyl group, a furylalkenyl group, an phenylalkenyl group, an phenylalkynyl group, a biphenyllylalkyl group, a biphenyllylalkenyl group; the aromatic group includes phenyl group, biphenyl group, naphthyl group, thienyl group or furyl group; which araliphatic groups and aromatic groups may optionally be substituted once, twice or three times by alkyl groups having 1 to 12 carbon atoms, alkenyl groups having 2 to 12 carbon atoms, alkynyl groups having 2 to 12 carbon atoms, alkoxyl groups having 1 to 12 carbon atoms, alkenyloxyl groups having 1 to 12 carbon atoms, alkylnyloxyl groups having 2 to 12 carbon atoms, methylenedioxy group, ethylenedioxy group, aralkyl groups having 7 to 12 carbon atoms, aralkyloxyl groups having 7 to 12 carbon atoms, phenoxyl group, alkyloxycarbonyl groups having 2 to 9 carbon atoms, alkylcarbonyloxyl groups having 2 to 9 carbon atoms and halogen atoms; which R may have one or more substituents of a hologen atom, an alkyl group having 1 to 10 carbon atoms, an alkenyl group having 2 to 10 carbon atoms, an alkynyl group having 2 to 10 carbon atoms, an alkenyloxyl group having 2 to 10 carbon atoms, an alkynyloxyl group having 2 to 10 carbon atoms, an alkoxyl group having 1 to 10 carbon atoms, an alkanoyloxyl group having 1 to 10 carbon atoms, an aralkanoyloxyl group having 8 to 13 carbon atoms, an alkoxycarbonyl group having 2 to 11 carbon atoms, an aralkoxycarbonyl group having 8 to 13 carbon atoms, an alkanoyl group having 2 to 12 carbon atoms, an aralkanoyl group having 8 to 13 carbon atoms, an alkanoylalkoxyl group having 3 to 13 carbon atoms, alkoxyalkoxyl group having 2 to 12 carbon atoms, methylendioxy group, ethylenedioxy group, an alkylthio group having 1 to 10 carbon atoms, an alkanoylthio group having 2 to 10 carbon atoms, an alkanoylamino group having 2 to 10 carbon atoms, an alkylaminocarbonyl group having 2 to 10 carbon atoms, a dialkylaminocarbonyl group having 3 to 12 carbon atoms, an aralkyl group having 7 to 15 carbon atoms, an aralkyloxyl group having 7 to 15 carbon atoms, a dialkylamino group having 2 to 8 carbon atoms, or phenoxyl group; X is hydrogen, chlorine or bromine atom, and Y is chlorine or bromine atom; or with
(b) a carboxylic acid represented by the formula (III)

   RCOOH (III)

   wherein R has the same meaning as defined above, in the presence of an acid anhydride represented by the formula (IV)

   (XYCHCO)₂O (IV)

   wherein X and Y have the same meanings as defined above.

The reaction is conducted usually in an solvent for which such halogenated hydrocarbons as carbon tetrachloride, tetrachloroethylene and tetrachloroethane are preferably used. The solvent may be employed alone or in a mixture of more than two solvents, and the amount of solvent is not critical.

As the catalyst, boron trifluoride or a boron trifluoride complex is used, and the boron trifluoride complex includes boron trifluoride-diethyl ether complex, boron trifluoride-methanol complex, boron trifluoride-acetic acid complex, and the like.

Aromatic compounds employed as raw materials of the present invention are those applicable to Friedel-Crafts Reaction, which are exemplified as follows;
(a) benzene, biphenyl, terphenyl, naphthalene and anthracene, in which benzene, biphenyl and terphenyl are substituted once to four times by a substituent containing at least one electron-donative group, and naphthalene and anthracene are unsubstituted or substituted once to four times by substitutents containing at least one electron-donative group,
(b) a thiophene derivative having not more than three substituents,
(c) pyrrol and a pyrrole derivative having a substituent at the nitrogen atom and/or having substituents of not more than two at the carbon atom,
(d) a furan derivative represented by the formula 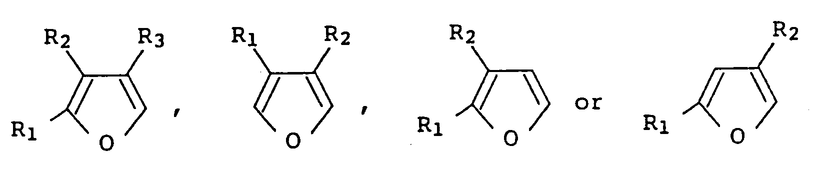 wherein R₁, R₂ or R₃ denotes a substituent,
   except the compounds represented by the formula 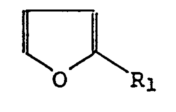 wherein R1 = H or lower alkyl group,
(e) a heteroatom-containing aromatic compound represented by the formula 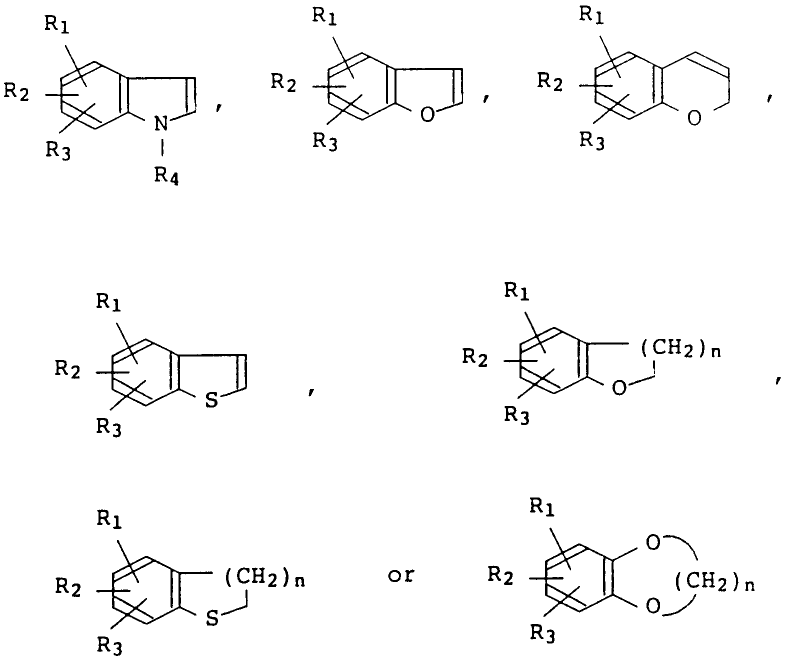 wherein R₁, R₂ or R₃ denotes a substituent, R₄ denotes hydrogen atom, a lower alkyl or an acyl group, and n denotes an integer of 1 to 4, and
(f) a condensed ring aromatic compound represented by the formula 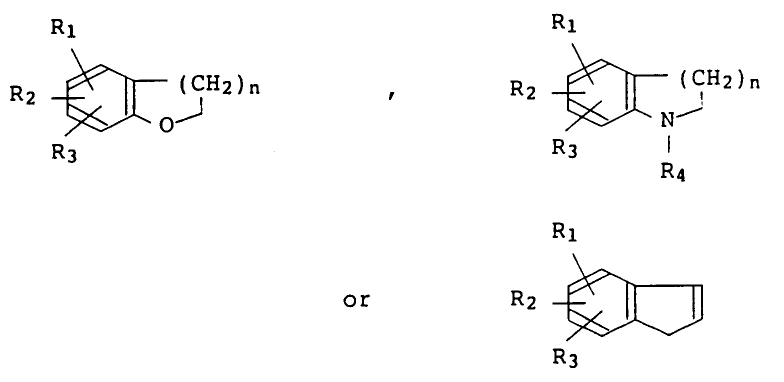 wherein R₁, R₂ or R₃ denotes a substituent, R₄ denotes a lower alkyl group or an acyl group with the proviso that at least one of substituent is a "electron-donative group", and n denotes an integer of 1 to 4.

In the above explanation on the aromatic compound, the term "electron-donative group" implies the following group;
(a) an alkyloxy group, alkenyloxy group and alkynyloxy group having respectively a straight chained or branched C₁-C₁₂ alkyl, alkenyl or alkynyl group,
(b) a straight chained or branched C₁-C₁₂ alkanoyloxy group,
(c) a straight chained or branched C₈-C₁₅ aralkanoyloxy group,
(d) a substituted or unsubstituted C₇-C₁₈ aralkyloxy group,
(e) an alkylthio group and alkylcarbonylthio group having C₁-C₁₂ alkyl,
(f) an alkylcarbonylalkoxy group having 3 to 12 carbon atoms,
(g) an alkylcarbonyloxyalkyl group having 3 to 12 carbon atoms,
(h) an alkyloxyalkoxy group having 2 to 12 carbon atoms,
(i) an alkyloxycarbonylalkoxy group having 3 to 12 carbon atoms,
(j) an alkylcarbonyloxyalkoxy group having 3 to 12 carbon atoms,
(k) a halogen atom,
(l) methylenedioxy, ethylenedioxy and phenoxy,
(m) a dialkylamino, an alkylamino, pyridino, pyrimidino, morpholino and N-(lower)alkylpyrrolidino.

Furthermore, the substituent includes the above "electron-donative group" and the following group;
(a) C₁-C₁₂ alkyl, C₂-C₁₂ alkenyl and C₂-C₁₂ alkynyl,
(b) C₇-C₁₈ aralkyl with/without substituent,
(c) an alkanoylalkyl and alkoxycarbonylalkyl group having 3 to 12 carbon atoms,
(d) C₁-C₁₂ acylamino,
(e) an alkoxyalkyl group having 2 to 12, and
(f) an aralkylcarbonyloxyalkyl group having 9 to 15 carbon atoms.

In the process, though the amount of catalyst to be used is generally from 0.05 to 1 equivalents based on the mixed acid anhydride (II), the reaction proceeds even in an amount of catalyst of from 0.05 to 0.2. The amount of aromatic compound is 1.0 or more equivalents, preferably from 1.2 to 2 equivalents based on the mixed acid anhydride (II), however, an increased amount of the mixed acid anhydride may can be employed in case of the aromatic compound is more expensive. The reaction temperature is in a range of generally from -5 to 120°C, preferably from 20 to 90°C. The reaction time is generally in a range of from 0.5 to 20 hours, but it is not particularly limited.

The mixed acid anhydride represented by the formula (II), which is one of starting materials for the above-mentioned reaction, can be produced by reacting the above-mentioned carboxylic acid RCOOH (III) with a haloacetic acid compound represented by the formula (V)

XYCHCOZ (V)

wherein Z is hydroxyl group, chlorine or bromine atom, and X and Y are as defined above.

The carboxylic acid represented by the formula (III) may include their optical isomers, and they are exemplified by acetic acid, propionic acid, butyric acid, isobutyric acid, valeric acid, isovaleric acid, hexanoic acid, heptanoic acid, octanoic acid, lauric acid, decanoic acid, stearic acid, β-halopropionic acid (halo denotes fluorine, chlorine, bromine or iodine atom), β-halopropionic acid, γ-halovaleric acid, 2-methylpropionic acid, 2-methylhexanoic acid, 2-methylocatnoic acid, 2-halo-3-methylpentanoic acid, S(+)-2-methylbutanoic acid, acrylic acid, crotonic acid, trans-2-pentenoic acid, 4-pentenoic acid, tarns-2-hexenoic acid, trans-3-hexenoic acid, cis-3-hexenoic acid, trans-2-methyl-2-pentenoic acid, 4-pentynoic acid, 2-octenoic acid, 2-octyonoic acid, ω-halo-5-cis-heptenoic acid, undecylenic acid, oleic acid, farnesyl acetic acid, cyclopropane carboxylic acid, (+)-2,2-dimethylcyclopropane carboxylic acid, cyclopentane carboxylic acid, cyclohexane carboxylic acid, methylcyclopentane carboxylic acid, 2-cyclohexene-1-carboxylic acid, cyclohexyl acetic acid, cyclohexyl butyric acid, benzoic acid, 4-methylbenzoic acid, 2,4-dimethylbenzoic acid, 4-methoxybenzoic acid, 4-octyloxybenzoic acid, 2,4-dichlorobenzoic acid, 4-bromobenzoic acid, 3,4-dimethoxybenzoic acid, 3,4-methylenedioxybenzoic acid, 3,4-dibenzyloxybenzoic acid, 2,4-dichlorobenzoic acid, 3,4,5-trimethoxybenzoic acid, 4-acetyloxybenzoic acid, 3,4-diacetyloxybenzoic acid, 4-acetylbenzoic acid, 4-propionylbenzoic acid, 4-methoxycarbonylbenzoic acid, 3-ethoxycarbonylbenzoic acid, thiophene carboxylic acid, phenyl acetic acid, 3-phenyl propionic acid, phenoxy acetic acid, 4-chlorophenyl acetic acid, α-chlorophenyl acetic acid, 4-chloro-α-isopropylphenyl acetic acid, 4-methylphenyl acetic acid, 4-methoxyphenyl acetic acid, α-phenyl propionic acid, 2-thienyl acetic acid, 4-(2-thienyl) butyric acid, 3-methoxy propionic acid, 5-ethoxy pentanoic acid, butoxy pentanoic acid, 6-methoxy hexanoic acid, monomethyl succinate, monomethyl glutarate, monomethyl suberate, levulinic acid, β-acetyloxy propionic acid, 10-oxo undecanoic acid, biphenylyl acetic acid, naphthalene carboxylic acid, furan carboxylic acid and 4′-octyloxy-4-carboxylbiphenyl.

When a haloacetic acid of the formula (V) in which substituent Z is hydroxyl group is used as the haloacetic acid compound, the aimed mixed acid anhydride can be obtained by the dehydration reaction in the presence or absence of a dehydrating agent, however, the aimed compound is easily available by the reaction using a haloacetyl halide in which the substituent Z is chlorine or bromine atom in the presence of an organic base and in a solvent. In the latter reaction, the amount of haloacetyl halide is generally from 1 to 1.5 equivalents based on the carboxylic acid. The organic base used in this reaction includes triethylamine, pyridine, diethylaniline and the like, and the amount thereof is generally from 1 to 1.5 equivalents based on the carboxylic acid. The reaction temperature is generally in a range of from -20 to 50°C. The reaction time is generally in a range of from 0.5 to 10 hours, but it is not particularly limited.

The reaction mixture obtained is subjected to filtration for separating off the resulting hydrochloride or hydrobromide salt of organic base to obtain crude solution of the mixed acid anhydride, which is then purified to obtain the mixed acid anhydride. The crude solution mentioned above can be used as it is for the next reaction with a aromatic compound. Accordingly, it is advantageous to use a solvent same as the solvent used in the next step.

In the case of the process (b) mentioned above, same carboxylic acids as exemplified for carboxylic acids of (III) may be used, and the acid anhydride (IV) includes, for example, chloroacetic anhydride, bromoacetic anhydride, dichloroacetic anhydride and the like. The amount of aromatic compound is 1.0 equivalent or more, preferably from 1.2 to 1.5 equivalents based on the carboxylic acid (III), however, an increased amount of the carboxylic acid may be employed in case of the aromatic compound is more expensive. The amount of acid anhydride (IV) is 1.0 equivalent or more, preferably from 1.1 to 1.3 equivalents based on the carboxylic acid (III). Although the amount of the boron trifluoride or boron trifluoride complex catalyst is generally from 0.02 to 0.2 equivalents based on the carboxylic acid (III), this amount is not restrictive and may be used in an amount of more than the above-mentioned amount. The reaction temperature is generally in a range of from -5 to 150°C, preferably from 20 to 90°C. The reaction time is generally in a range of from 0.5 to 20 hours, but it is not particularly limited.

The reaction mixture thus obtained is post-treated in usual manners and then purified by methods such as distillation, column chromatography or the like, if necessary, to obtain in high yield the aimed acylated derivatives of aromatic compounds.

Thus, according to the present invention, aimed acylaromatic compounds can be produced advantageously for the industry from a aromatic compound and a mixed acid anhydride (II) or a carboxylic acid (III).

Moreover, since boron trifluoride, boron trifluoride complexes and acid anhydrides are commercially available cheeply, industrial value of the present invention is enhanced further.

The present invention will be illustrated in more detail below by way of showing Examples.

### Example 1

In 50 ml of dichloroethane were dissolved 9.41 g (0.05 mole) of suberic acid monomethyl ester and 9.83 g (0.0575 mole) of monochloroacetic anhydride. To the resulting solution were added 5.47 g (0.065 mole) of thiophene and 0.71 g of boron trifluoride-diethyl ether complex and the resulting mixture was then stirred at 60°C for 5 hours. After completion of the reaction, the reaction solution was cooled and washed successively with water, 5% aqueous sodium carbonate solution and water. The organic layer was dried over anhydrous magnesium sulfate and then concentrated under reduced pressure to obtain 11.5 g of 2-(7-methoxycarbonyl-1-oxoheptyl)thiophene.
Yield: 91%

### Example 2

Reaction and post-treatment were carried out in the same manner as in Example 1, except that 6.38 g (0.065 mole) of 2-methylthiophene and 6.4 g (0.05 mole) of cyclohexane carboxylic acid were substituent for the thiophene and the suberic acid monomethyl ester, to obtain 8.3 g of 2-cyclohexylcarbonyl-5-methylethiophene.
Yield: 80%

### Example 3

Reaction and post-treatment were carried out in the same manner as in Example 1, except that 10.6 g (0.065 mole) of 2-bromothiophene and 6.1 g (0.05 mole) of benzoic acid were substituent for the thiophene and the suberic acid monomethyl ester, to obtain 6.95 g of 2-benzoyl-5-bromothiophene.
Yield: 52%

### Example 4

In 40 ml of dichloroethane were dissolved 6.51 g (0.05 mole) of heptanoic acid and 10.26 g (0.06 mole) of monochloroacetic anhydride. To the resulting solution were added 7.57 g (0.07 mole) of anisole and 0.9 g of boron trifluoride-acetic acid complex and the resulting mixture was then stirred at refluxing for 8 hours. After completion of the reaction, the reaction solution was cooled and washed successively with water, 5% aqueous sodium carbonate solution and water. The organic layer was concentrated under reduced pressure to obtain 9.25 g of 4-methoxy-1-heptanoyl-benzene.
Yield: 84%

### Example 5

In 50 ml of dichloroethane were dissolved 5.8 g (0.05 mole) of levulinic acid and 10.26 g (0.06 mole) of monochloroacetic anhydride. To the resulting solution were added 8.98 g (0.065 mole) of 1,2-di-methoxybenzene and 0.71 g of boron trifluoride-diethyl ether complex and the resulting mixture was then stirred at 50°C for 5.5 hours. After completion of the reaction, the reaction solution was cooled and washed successively with water, 5% aqueous sodium carbonate solution and water. The organic layer was dried over anhydrous magnesium sulfate and then concentrated under reduced pressure to obtain 9.66 g of 4-(β-acetylpropionyl)-1,2-dimethoxybenzene.
Yield: 76%

### Examples 6 to 10

Reaction and post-treatment were carried out in the same manner as in Example 1, except that each carboxylic acid (0.05 mole) shown in Table 1 was a substituent for suberic acid monomethyl ester in Example 1, to obtain results shown in Table 1.

**Table 1**

| Example No. | Starting carboxylic acid | | Acylated product | | |
|---|---|---|---|---|---|
| | Name of compound | Amount used | Amount produced | Yield | Name of compound |
| 6 | 4-chlorophenyl acetic acid | 8.53^{(g)} | 11.4^{(g)} | 96^{(%)} | 2-(4-chlorophenyl)acetylthiophene |
| 7 | cyclohexyl butyric acid | 6.51 | 11 | 93 | 2-(cyclohexylbutyryl) thiophene |
| 8 | 4-pentic acid | 4.9 | 6.65 | 81 | 2-(4-pentynoyl) thiophene |
| 9 | trans-3-hexenic acid | 5.7 | 7.75 | 86 | 2-(trans-3-hexenoyl)thiophene |
| 10 | 2-thienyl acetic acid | 7.11 | 9.37 | 90 | 2-(2-thienylacetyl) thiophene |

### Example 11

In 50 ml of dichloroethane were dissolved 3.7 g (0.05 mole) of propionic acid and 10.26 g (0.06 mole) of monochloroacetic anhydride. To the resulting solution were added 5.36 g (0.065 mole) of octyloxybenzene and 0.71 g of boron trifluoride-diethyl ether complex and the resulting mixture was then stirred at 70°C for 5 hours. After completion of the reaction, the reaction solution was cooled and washed successively with water, 5% aqueous sodium carbonate solution and water. The organic layer was dried over anhydrous magnesium sulfate and then concentrated under reduced pressure to obtain 7.2 g of 4-octyloxypropiophenone.
Yield: 55%

### Example 12

In 50 ml of dichloroethane were dissolved 6.51 g (0.05 mole) of heptanoic acid and 10.26 g (0.06 mole) of monochloroacetic anhydride. To the resulting solution were added 4.36 g (0.065 mole) of pyrrole and 0.71 g of boron trifluoride-diethyl ether complex, and the resulting mixture was then stirred at 50°C for 5.5 hours. After completion of the reaction, the reaction solution was cooled and washed successively with water, 5% aqueous sodium carbonate solution and water. The organic layer was dried over anhydrous magnesium sulfate and then concentrated under reduced pressure to obtain 5.74 g of 2-heptanoyl-pyrrole.
Yield: 64%

### Example 13

In 50 ml of tetrachloroethane were dissolved 5.1 g (0.05 mole) of 2S-methyl butyric acid and 14.39 g (0.06 mole) of dichloroacetic anhydride. To the resulting solution were added 10.92 g (0.065 mole) of 1,2,3-trimethoxybenzene and 0.71 g of boron trifluoridediethyl ether complex and the resulting mixture was then stirred at 50°C for 3 hours. After completion of the reaction, the reaction solution was cooled and washed successively with water, 5% aqueous sodium carbonate solution and water. The organic layer was dried over anhydrous magnesium sulfate and then concentrated under reduced pressure to obtain 10.1 g of 1,2,3-trimethoxyphenyl(1-methylpropyl)ketone.
Yield: 80%

### Example 14

In 50 ml of dichloroethane were dissolved 8.51 g (0.05 mole) of 4-(2-thienyl)butyric acid and 14.39 g (0.06 mole) of dichloroacetic anhydride. To the resulting solution was added 0.065 mole of 3,5-dimethylfuran and 0.71 g of boron trifluoride-diethyl ether complex and the resulting mixture was then stirred at 50°C for 7 hours. After completion of the reaction, the reaction solution was cooled and washed successively with 5% aqueous sodium carbonate solution and water. The organic layer was concentrated under reduced pressure to obtain 2-[4-(2-thienyl)butyryl]3,5-dimethylfuran.
Yield: 90%

### Example 15

In 50 ml of dichloroethane were dissolved 7.51 g (0.05 mole) of β-phenylpropionic acid and 9.83 g (0.0575 mole) of monochloroacetic anhydride. To the resulting solution were added 8.17 g (0.06 mole) of phenylacetate and 1.0 g of boron trifluoride-acetic acid complex and the resulting mixture was then stirred at 90°C for 8 hours. After completion of the reaction, the reaction solution was cooled and washed successively with water, 5% aqueous sodium carbonate solution and water. The organic layer was dried over anhydrous magnesium sulfate and then concentrated under reduced pressure to obtain 10.7 g of 4-acetyloxy-1-(β-phenyl)propionylbenzene.
Yield: 80%

### Example 16

In 50 ml of dichloroethane were dissolved 7.31 g (0.05 mole) of 6-methoxy hexanoic acid and 14.39 g (0.06 mole) of dichloroacetic anhydride. To the resulting solution were added 7.93 g (0.065 mole) of methylenedioxybenzene and 0.71 g of boron trifluoridediethyl ether complex and the resulting mixture was then stirred at 70°C for 6 hours. After completion of the reaction, the reaction solution was cooled and washed with 5% aqueous sodium carbonate solution and water in this order. The organic layer was concentrated under reduced pressure to obtain 11.2 g of 4-(6-methoxyhexanoyl)-1,2-methylenedioxybenzene.

### Example 17

In 50 ml of toluene were dissolved 4.31 g (0.05 mole) of crotonic acid, 5.47 g (0.065 mole) of thiophene and 10.26 g (0.06 mole) of monochloroacetic anhydride. To the resulting solution was added 0.71 g of boron trifluoride-diethyl ether complex and the resulting mixture was stirred at 50°C for 6.5 hours. After completion of the reaction, the reaction solution was cooled and washed with 5% aqueous sodium carbonate solution and water in this order. The organic layer was concentrated under reduced pressure to obtain 4.26 g of 2-crotonylthiophene.
Yield: 56%

### Example 18

Reaction and post-treatment were carried out in the same manner as in Example 12, except that 5.27 g (0.065 mole) of 1-methylpyrrole and 4.4 g (0.05 mole) of isobutyric acid were substituent for the pyrrole and heptanoic acid, to obtain 6 g of 1-methyl-2-isobutyrylpyrrole.
Yield: 80%

### Examples 19 to 22

Reaction and post-treatment were carried out in the same manner as in Example 1, except that each carboxylic acid shown in Table 2 was a substituent for suberic acid monomethyl ester in Example 1, to obtain results shown in Table 2.

**Table 2**

| Example No. | Starting carboxylic acid | | Acylated product | | |
|---|---|---|---|---|---|
| | Name of compound | Amount used | Amount produced | Yield | Name of compound |
| 19 | 4-methyl benzoic acid | 6.81^{(g)} | 5.26^{(g)} | 52^{(%)} | 4-methylbenzoyl thiophene |
| 20 | 4-bromo benzoic acid | 10.01 | 5.88 | 44 | 4-bromobenzoyl thiophene |
| 21 | thiophene carboxylic acid | 6.41 | 5.73 | 59 | 2,2-dithienyl ketone |
| 22 | cyclopropane carboxylic acid | 4.3 | 6.09 | 80 | cyclopropyl-2-thienyl ketone |

### Example 23

Reaction and post-treatment were carried out in the same manner as in Example 14, except that 10-acetyloxydecanoic acid was a substituent for 4-(2-thienyl)butyric acid, to obtain 2-(10-acetyloxydecanoyl)-3,5-dimethylfuran.
Yield: 90%

### Example 24

Reaction and post-treatment were carried out in the same manner as in Example 15, except that 5.71 g (0.05 mole) of (+)-2,2-dimethylcyclopropanecarboxylic acid was a substituent for β-phenylpropionic acid, to obtain 8.83 g of acetyloxyphenyl-2,2-dimethylcyclopropylketone.
Yield: 76%

### Examples 25 to 26

Reaction and post-treatment were carried out in the same manner as in Example 1, except that each carboxylic acid shown in Table 3 was a substituent for suberic acid monomethyl ester in Example 1, to obtain results shown in Table 3.

**Table 3**

| Example No. | Starting carboxylic acid | | Acylated product | | |
|---|---|---|---|---|---|
| | Name of compound | Amount used | Amount produced | Yield | Name of compound |
| 25 | β-chloropropionic acid | 5.43^{(g)} | 4.63^{(g)} | 53^{(%)} | 2-(β-chloropropionyl)thiophene |
| 26 | stearic acid | 14.2 | 16.3 | 93 | 2-(octadecanoyl)thiophene |

### Example 27

In 50 ml of tetrachloroethane were dissolved 6.51 g (0.05 mole) of heptanoic acid and 9.83 g (0.0575 mole) of monochloroacetic anhydride. To the resulting solution were added 12.74 g (0.06 mole) of 4-acetoxybiphenyl and 0.8 g of boron trifluoride-diethyl ether complex and the resulting mixture was then stirred at 100°C for 10 hours. After completion of the reaction, the reaction solution was cooled and washed with 5% aqueous sodium carbonate solution and water in this order. The organic layer was concentrated under reduced pressure to obtain 12.0 g of 4-acetoxy-4′-heptanoylbiphenyl.
Yield: 74%

### Example 28

In 50 ml of dichloroethane were dissolved 11.51 g (0.05 mole) of 10-acetyloxydecanoic acid and 14.39 g (0.06 mole) of dichloroacetic anhydride. To the resulting solution were added 9.89 g (0.065 mole) of 3,4-dimethoxytoluene and 0.71 g of boron trifluoridediethyl ether complex and the resulting mixture was then stirred at 75°C for 5 hours. After completion of the reaction, the reaction solution was cooled and washed successively with water, 5% aqueous sodium carbonate solution and water. The organic layer was dried over anhydrous magnesium sulfate and then concentrated under reduced pressure to obtain 15.3 g of 3,4-dimethoxy-6-(10-acetyloxydecanoyl)toluene.
Yield: 84%

### Example 29

In 50 ml of tetrachloroethane were dissolved 4.41 g (0.05 mole) of butyric acid and 9.83 g (0.0575 mole) of monochloroacetic anhydride. To the resulting solution were added 7.69 g (0.06 mole) of naphthalene and 0.9 g of boron trifluoride-diethyl ether complex and the resulting mixture was then stirred at 115°C for 8 hours. After completion of the reaction, the reaction solution was cooled and washed successively with water, 5% aqueous sodium carbonate solution and water. The organic layer was dried over anhydrous magnesium sulfate and then concentrated under reduced pressure to obtain 4.26 g of butyrylnaphthalene.
Yield: 43%

### Example 30

In 50 ml of dichloroethane were dissolved 10.6 g (0.05 mole) of 3,4,5-trimethoxybenzoic acid and 9.83 g (0.0575 mole) of monochloroacetic anhydride. To the resulting solution were added 5.47 g (0.065 mole) of thiophene and 0.71 g of boron trifluoride-diethyl ether complex and the resulting mixture was then stirred at 65°C for 6 hours. After completion of the reaction, the reaction solution was cooled and washed successively with water, 5% aqueous sodium carbonate solution and water. The organic layer was dried over anhydrous magnesium sulfate and then concentrated under reduced pressure to obtain 5.77 g of 2-(3,4,5-trimethoxybenzoyl)thiophene.
Yield: 41.5%

### Example 31

In 50 ml of dichloroethane were dissolved 10.5 g (0.05 mole) of 11-cyanoundecanoic acid and 10.26 g (0.06 mole) of monochloroacetic anhydride. To the resulting solution were added 8.98 g (0.065 mole) of 1,2-dimethoxybenzene and 0.71 g of boron trifluoridediethyl ether complex and the resulting mixture was then stirred at 75°C for 6 hours. After completion of the reaction, the reaction solution was cooled and washed successively with water, 5% aqueous sodium carbonate solution and water. The organic layer was dried over anhydrous magnesium sulfate and then concentrated under reduced pressure to obtain 14.2 g of 4-(11-cyanoundecanoyl)-1,2-dimethoxybenzene.
Yield: 86%

### Example 32

In 50 ml of dichloroethane were dissolved 6.31 g (0.05 mole) of 1-cyclohexene-1-carboxylic acid and 10.26 g (0.06 mole) of monochloroacetic anhydride. To the resulting solution were added 8.98 g (0.065 mole) of 1,2-dimethoxybenzene and 0.71 g of boron trifluoridediethyl ether complex and the resulting mixture was then stirred at 75°C for 6 hours. After completion of the reaction, the reaction solution was cooled and washed successively with water, 5% aqueous sodium carbonate solution and water. The organic layer was dried over anhydrous magnesium sulfate and then concentrated under reduced pressure to obtain 7.27 g of 4-(1-cyclohexenoyl)-1,2-dimethoxybenzene.
Yield: 59%

### Example 33

In 50 ml of tetrachloroethane were dissolved 12.5 g (0.05 mole) of 4-octyloxybenzoic acid and 14.39 g (0.06 mole) of dichloroacetic anhydride. To the resulting solution were added 11.1 g (0.06 mole) of benzylphenyl ether and 0.71 g of boron trifluoride-diethyl ether complex and the resulting mixture was then stirred at 105°C for 7 hours. After completion of the reaction, the reaction solution was cooled and washed successively with water, 5% aqueous sodium carbonate solution and water. The organic layer was dried over anhydrous magnesium sulfate and then concentrated under reduced pressure to obtain 13.74 g of 4-(4-octyloxybenzoyl)phenylbenzyl ether.
Yield: 66%

### Example 34

In 50 ml of tetrachloroethane were dissolved 5.11 g (0.05 mole) of valeric acid and 9.83 g (0.0575 mole) of monochloroacetic anhydride. To the resulting solution were added 9.42 g (0.06 mole) of bromobenzene and 0.8 g of boron trifluoride-diethyl ether complex and the resulting mixture was then stirred at 115°C for 10 hours. After completion of the reaction, the reaction solution was cooled and washed successively with water, 5% aqueous sodium carbonate solution and water. The organic layer was dried over anhydrous magnesium sulfate and then concentrated under reduced pressure to obtain 6.75 g of valerylbromobenzene.
Yield: 56%

### Example 35

Reaction and post-treatment were carried out in the same manner as in Example 1, except that 9.71 g (0.05 mole) of 4-acetoxyphenylacetic acid was a substituent for suberic acid monomethyl ester, to obtain 10.0 g of 2-(4-acetoxyphenylacetyl)thiophene.
Yield: 77%

### Example 36

A four neck flask equipped with a stirrer and a thermometer was charged with 9.41 g (0.05 mole) of suberic acid monomethyl ester, 5.11 g (0.0505 mole) of triethylamine and 60 ml of carbon tetrachloride. Under stirring, 5.71 g (0.0505 mole) of monochloroacetyl chloride was added dropwise thereto at 0° to 5°C. After completion of dropping, the reaction was continued at room temperature for 3 hours. After completion of the reaction, the reaction mixture was filtered under reduced pressure to remove formed hydrochloride salt of triethylamine.

To the filtrate were added 0.075 mole of thiophen and 0.71 g (0.005 mole) of boron trifluoridediethyl ether complex and the resulting mixture was heated at 40°C for 4 hours. After completion of the reaction, the reaction solution was cooled and washed successively with 50 ml of water, 50 ml of 5% aqueous sodium carbonate solution and 50 ml of water. The organic layer was dried over anhydrous magnesium sulfate and then concentrated under reduced pressure to obtain 2-(7-methoxycarbonyl-1-oxoheptyl)thiophen.
Yield: 82%

### Example 37

A four neck flask equipped with a stirrer and a thermometer was charged with 3.7 g (0.05 mole) of propionic acid, 5.11 g (0.050 mole) of triethylamine and 60 ml of carbon tetrachloride. Thereto was added dropwise 0.0505 mole of dichloroacetyl chloride at 0° to 5°C. After completion of dropping, the reaction mixture was filtered under reduced pressure to remove formed hydrochloride salt of triethylamine.

To the filtrate were added 0.065 mole of 3,5-dimethylfuran and 1.0 g of boron trifluoride-acetic acid complex and the resulting mixture was stirred at 50°C for 6 hours. After completion of the reaction, post-treatment was carried out in the same manner as in Example 1 to obtain 2-propionyl-3,5-dimethylfuran.
Yield: 41.5%

### Examples 38 - 45

Reactions and post-treatments were carried out in the same manner as in Example 28, except that each carboxylic acid shown in Table 4 was a substituent for 10-acetyloxydecanoic acid to obtain results shown in Table 4.

**Table 4**

| Example No. | Starting carboxylic acid | Acylated product |
|---|---|---|
| 38 | 4-(1-cyclohexenyloxy)benzoic acid | 3,4-dimethoxy-6-[4-(1-cyclohexenyloxy)benzoyl]toluene |
| 39 | 4-phenylbutanoic acid | 3,4-dimethoxy-6-(4-phenylbutanoyl)toluene |
| 40 | 4-biphenylcarboxylic acid | 3,4-dimethoxy-6-(4-biphenylcarbonyl)-toluene |
| 41 | 3-furyl-propionic acid | 3,4-dimethoxy-6-(3-furylpropanoyl)-toluene |
| 42 | 4-methylthiobenzoic acid | 3,4-dimethoxy-6-(4-methylthiobenzoyl)-toluene |
| 43 | 3-(benzoyl)-propionic acid | 3,4-dimethoxy-6-[3-(benzoyl)propanoyl]-toluene |
| 44 | 3,4-methylenedioxybenzoic acid | 3,4-dimethoxy-6-(3,4-methylenedioxybenzoyl)-toluene |
| 45 | 3-methylthiopropionic acid | 3,4-dimethoxy-6-(3-methylthiopropanoyl)-toluene |

### Examples 46 - 50

Reactions and post-treatments were carried out in the same manner as in Example 31, except that each carboxylic acid shown in Table 5 was a substituent for 11-cyanoundecanoic acid, to obtain results shown in Table 5.

**Table 5**

| Example No. | Starting carboxylic acid | Acylated product |
|---|---|---|
| 46 | 3-acetylthiopropionic acid | 4-(3-acetylthiopropanoyl)-1,2-dimethoxybenzene |
| 47 | cyclohexylpropenoic acid | 4-(cyclohexylpropenoyl)-1,2-dimethoxybenzene |
| 48 | 1-naphthylacetic acid | 4-(1-naphthylaceto)-1,2-dimethoxybenzene |
| 49 | 4-benzyloxybenzoic acid | 4-(4-benzyloxybenzoyl)-1,2-dimethoxybenzene |
| 50 | 2-furoic acid | 4-(2-furoyl)-1,2-dimethoxybenzene |

### Examples 51 - 52

Reactions and post-treatments were carried out in the same manner as in Example 12, except that each aromatic compound shown in Table 6 was a substituent for pyrrole to obtain results shown in Table 6.

**Table 6**

| Example No. | Starting aromatic compound | Acylated product |
|---|---|---|
| 51 | benzothiophene | 2-heptanoylbenzothiophene |
| 52 | benzofuran | 2-heptanoylbenzofuran |

## Claims

1. A process for producing an acylaromatic compound represented by the formula 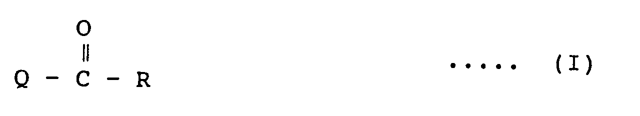 wherein Q is an aromatic compound residue and R is a straight, branched or cyclic aliphatic group, aromatic group or araliphatic group,
which comprises a reaction, in the presence of boron trifluoride or a boron trifluoride complex catalyst, of an aromatic compound except the compound represented by the formula 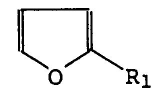 wherein R₁ = H or lower alkyl group,
either with
a) a mixed acid anhydride represented by the formula 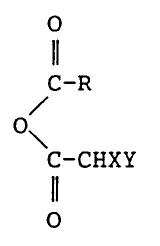 wherein R is a straight, branched or cyclic aliphatic group, aromatic or araliphatic group, which R may have one or more substituents of a halogen atom, an alkyl group having 1 to 10 carbon atoms, an alkenyl group having 2 to 10 carbon atoms, an alkynyl group having 2 to 10 carbon atoms, an alkenyloxyl group having 2 to 10 carbon atoms, an alkynyloxyl group having 2 to 10 carbon atoms, an alkoxyl group having 1 to 10 carbon atoms, an alkanoyloxyl group having 1 to 10 carbon atoms, an aralkanoyloxyl group having 8 to 13 carbon atoms, an alkoxycarbonyl group having 2 to 11 carbon atoms, an aralkoxycarbonyl group having 8 to 13 carbon atoms, an alkanoyl group having 2 to 12 carbon atoms, an aralkanoyl group having 8 to 13 carbon atoms, an alkanoylalkoxyl group having 3 to 13 carbon atoms, alkoxyalkoxyl group having 2 to 12 carbon atoms, methylendioxy group, ethylenedioxy group, an alkylthio group having 1 to 10 carbon atoms, an alkanoylthio group having 2 to 10 carbon atoms, an alkanoylamino group having 2 to 10 carbon atoms, an alkylaminocarbonyl group having 2 to 10 carbon atoms, a dialkylaminocarbonyl group having 3 to 12 carbon atoms, an aralkyl group having 7 to 15 carbon atoms, an aralkyloxyl group having 7 to 15 carbon atoms, a dialkylamino group having 2 to 8 carbon atoms, or phenoxyl group; X is hydrogen, chlorine or bromine atom, and Y is chlorine or bromine atom; or with
(b) a carboxylic acid represented by the formula
RCOOH
wherein R has the same meaning as defined above, in the presence of an acid anhydride represented by the formula (IV)
(XYCHCO)₂O
wherein X and Y have the same meanings as defined above.

2. A process according to claim 1, wherein the straight, branched or cyclic aliphatic group is an alkyl group having 1 to 18 carbon atoms, an alkenyl group having 2 to 18 carbon atoms, an alkynyl group having 2 to 18 carbon atoms, a cyclic alkyl group having 3 to 8 carbon atoms, a cyclic alkenyl group having 3 to 8 carbon atoms, a cyclic alkynyl group having 3 to 8 carbon atoms, a cycloalkylalkyl group having 3 to 15 carbon atoms, a cycloalkylalkenyl group having 3 to 15 carbon atoms, a cycloalkylalkynyl group having 3 to 15 carbon atoms, a cycloalkenyalkyl group having 3 to 15 carbon atoms, a cycloalkynylalkyl group having 3 to 15 carbon atoms or a cycloalkenylalkenyl group having 3 to 15 carbon atoms.

3. A process according to Claim 1, wherein the aromatic or araliphatic group is a thienylalkyl group, a thienylalkenyl group, an aralkyl group, a thienylalkynyl group, a furylalkyl group, a naphthylalkyl group, a furylalkenyl group, an aralkenyl group, an aralkynyl group, a biphenyllylalkyl group, a biphenyllylalkenyl group unsubstituted or substituted once, twice or three times by alkyl groups having 1 to 12 carbon atoms, alkenyl groups having 1 to 12 carbon atoms, alkynyl groups having 1 to 12 carbon atoms, alkoxyl groups having 1 to 12 carbon atoms, alkenyloxy groups having 1 to 12 carbon atoms, alkynyloxyl groups having 2 to 12 carbon atoms, methylenedioxy group, ethylenedioxy group, aralkyl groups having 7 to 12 carbon atoms, aralkyloxyl groups having 7 to 12 carbon atoms, phenoxyl group, alkyloxycarbonyl groups having 2 to 9 carbon atoms, alkylcarbonyloxyl groups having 2 to 9 carbon atoms and halogen atoms.

4. A process according to claim 1, wherein the aromatic group is phenyl, biphenyl, naphthyl, thienyl or furyl group unsubstituted or substituted once, twice or three times by alkyl groups having 1 to 12 carbon atoms, alkenyl groups having 1 to 12 carbon atoms, alkynyl groups having 1 to 12 carbon atoms, alkoxyl groups having 1 to 12 carbon atoms, alkenyloxy groups having 1 to 12 carbon atoms, alkynyloxyl groups having 2 to 12 carbon atoms, methylenedioxy group, ethylenedioxy group, aralkyl groups having 7 to 12 carbon atoms, aralkyloxyl groups having 7 to 12 carbon atoms, phenoxyl group, alkyloxycarbonyl groups having 2 to 9 carbon atoms, alkylcarbonyloxyl groups having 2 to 9 carbon atoms and halogen atoms.

5. A process according to Claim 1, wherein the aromatic compound is one to which Friedel-Crafts Reaction is applicable.

6. A process according to Claim 5, wherein the aromatic compound is selected from the group consisting of
(a) benzene, biphenyl, terphenyl, naphthalene and anthracene, in which benzene, biphenyl and terphenyl are substituted once to four times by a substituent containing at least one electron-donative group, and naphthalene and anthracene are unsubstituted or substituted once to four times by substituents containing at least one electron-donative group,
(b) a thiophene derivative having not more than three substituents,
(c) pyrrole and a pyrrole derivative having a substituent at the nitrogen atom and/or having substituents of not more than two at the carbon atom,
(d) a furan derivative represented by the formula 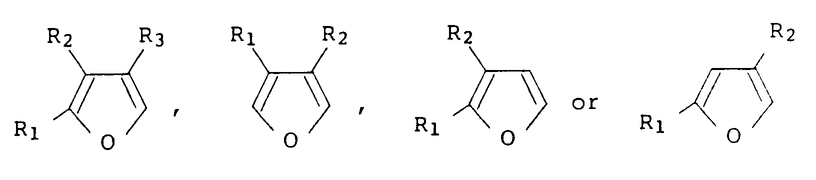 wherein R₁, R₂ or R₃ denotes a substituent,
(e) a heteroatom-containing aromatic compound represented by the formula 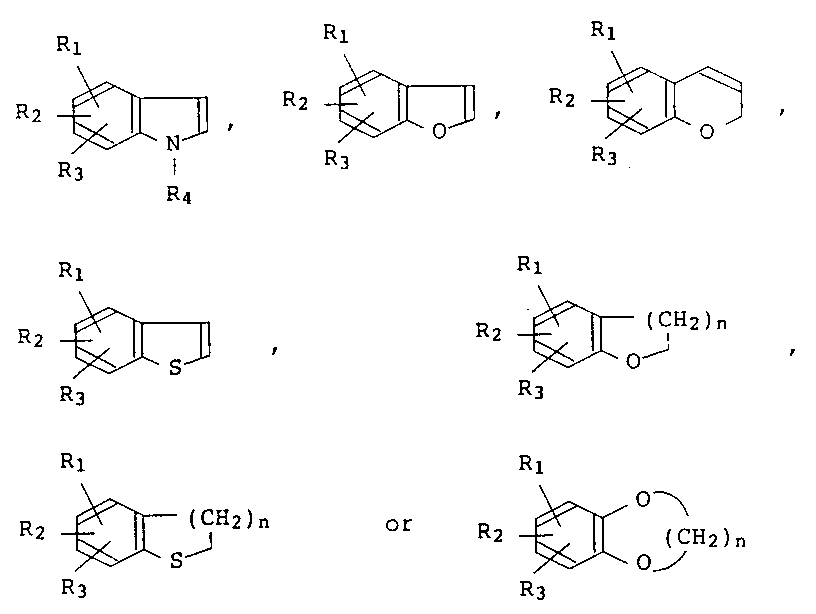 wherein R₁, R₂ or R₃ denotes a substituent, R₄ denotes hydrogen atom, a lower alkyl or an acyl group, and n denotes an integer of 1 to 4, and
(f) a condensed ring aromatic compound represented by the formula 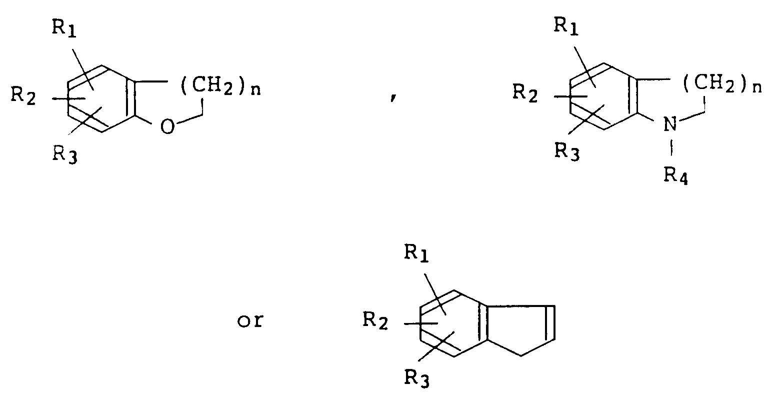 wherein R₁, R₂ or R₃ denotes a substituent, R₄ denotes a lower alkyl group or an acyl group with the proviso that at least one of substituent is a "electron-donative group", and n denotes an integer of 1 to 4.

7. A process according to Claim 6, wherein the electron-donative group is selected from the group consisting of
(a) an alkyloxy group, alkenyloxy group and alkynyloxy group having respectively a straight chained or branched C₁-C₁₂ alkyl, alkenyl or alkynyl group,
(b) a straight chained or branched C₁-C₁₂ alkanoyloxy group,
(c) a straight chained or branched C₈-C₁₅ aralkanoyloxy group,
(d) a substituted or unsubstituted C₇-C₁₈ aralkyloxy group,
(e) an alkylthio group and alkylcarbonylthio group having C₁-C₁₂ alkyl,
(f) an alkylcarbonylalkoxy group having 3 to 12 carbon atoms,
(g) an alkylcarbonyloxyalkyl group having 3 to 12 carbon atoms,
(h) an alkyloxyalkoxy group having 2 to 12 carbon atoms,
(i) an alkyloxycarbonylalkoxy group having 3 to 12 carbon atoms,
(j) an alkylcarbonyloxyalkoxy group having 3 to 12 carbon atoms,
(k) a halogen atom,
(l) methylenedioxy, ethylenedioxy and phenoxy,
(m) a dialkylamino, an alkylamino, pyridino, pyrimidino, morpholino and N-(lower)alkylpyrrolidino.

8. A process according to claim 6, wherein the substituent is selected from the group consisting of
(a) an alkyloxy group, alkenyloxy group and alkynyloxy group having respectively a straight chained or branched C₁-C₁₂ alkyl, alkenyl or alkynyl group,
(b) a straight chained or branched C₁-C₁₂ alkanoyloxy group,
(c) a straight chained or branched C₈-C₁₅ aralkanoyloxy group,
(d) a substituted or unsubstituted C₇-C₁₈ aralkyloxy group,
(e) an alkylthio group and alkylcarbonylthio group having C₁-C₁₂ alkyl,
(f) an alkylcarbonylalkoxy group having 3 to 12 carbon atoms,
(g) an alkylcarbonyloxyalkyl group having 3 to 12 carbon atoms,
(h) an alkyloxyalkoxy group having 2 to 12 carbon atoms,
(i) an alkyloxycarbonylalkoxy group having 3 to 12 carbon atoms,
(j) an alkylcarbonyloxyalkoxy group having 3 to 12 carbon atoms,
(k) a halogen atom,
(l) methylenedioxy, ethylenedioxy and phenoxy,
(m) a dialkylamino, an alkylamino, pyridino, pyrimidino, morpholino and N-(lower)alkylpyrrolidono,
(n) C₁-C₁₂ alkyl, C₁-C₁₂ alkenyl and C₁-C₁₂ alkynyl,
(o) C₇-C₁₈ aralkyl with/without substituent,
(p) an alkanoylalkyl and alkoxycarbonylalkyl group having 3 to 12 carbon atoms,
(q) C₁-C₁₂ acylamino,
(r) an alkoxyalkyl group having 2 to 12, and
(s) an aralkylcarbonyloxyalkyl group having 9 to 15 carbon atoms.

9. A process according to Claim 1, wherein the boron trifluoride complex is boron trifluoride-diethyl ether complex, boron trfifluoride-acetic acid complex or boron trifluoride-methanol complex.

10. A process according to Claim 1, wherein the amount of said catalyst is from 0.05 to 1 equivalents based on said mixed acid anhydride.

11. A process according to Claim 1, wherein the amount of said catalyst is from 0.02 to 0.2 equivalents based on said carboxylic acid.

12. A process according to Claim 1, wherein the amount of said aromatic compound is from 1.2 to 2 equivalents based on said mixed acid anhydride.

13. A process according to Claim 1, wherein the amount of said aromatic compound is from 1.2 to 2 equivalents based on said carboxylic acid.

14. A process according to Claim 1, wherein the amount of said acid anhydride is from 1.1 to 1.3 equivalents based on said carboxylic acid.

15. A process according to Claim 1, wherein said mixed acid anhydride represented by the formula 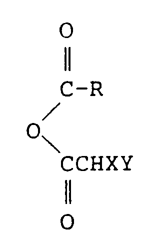 wherein R, X and Y are as defined above, is produced by reacting a carboxylic acid represented by the formula
RCOOH
wherein R is as defined above, with a haloacetic acid compound represented by the formula
XYCHCOZ
wherein X is hydrogen, chlorine or bromine atom, Y is bromine or chlorine atom, and Z is hydroxyl group, chlorine or bromine atom.

16. A process according to Claim 1 or 15, wherein said carboxylic acid is a saturated straight, branched or cyclic aliphatic carboxylic acid.

17. A process according to Claim 1 or 15, wherein said carboxylic acid is an unsaturated straight, branched or cyclic aliphatic carboxylic acid.

18. A process according to Claim 16, wherein said aliphatic carboxylic acid is a saturated alicyclic carboxylic acid having 4 to 9 carbon atoms.

19. A process according to Claim 1 or 15, wherein said carboxylic acid is benzoic acid.

20. A process according to Claim 1 or 15, wherein said carboxylic acid is benzoic acid substituted by one, two or three alkyl groups having 1 to 12 carbon atoms, alkoxyl groups having 1 to 12 carbon atoms or halogen atoms.

21. A process according to Claim 1 or 15, wherein said carboxylic acid is an aralkyl carboxylic acid.

22. A process according to Claim 1 or 15, wherein said carboxylic acid is a straight or branched aliphatic carboxylic acid having 2 to 19 carbon atoms substituted by alkoxyl group or alkoxycarbonyl group having 1 to 10 carbon atoms.

23. A process according to Claim 15, wherein the substituent Z of said haloacetic acid compound is chlorine or bromine atom.

24. A process according to Claim 23, wherein said reaction is carried out in the presence of an organic base.

25. A process according to Claim 24, wherein said organic base is triethylamine, pyridine or diethylaniline.

26. A process according to Claim 23, wherein the amount of said haloacetic acid compound is from 1 to 1.5 equivalents based on said carboxylic acid.

27. A process according to Claim 24, wherein the amount of said organic base is from 1 to 1.5 equivalents based on said carboxylic acid.

28. A process according to Claim 23, wherein the reaction temperature is in a range of from -20 to 50°C.

## Patentansprüche

1. Verfahren zur Herstellung einer acylaromatischen Verbindung der Formel: 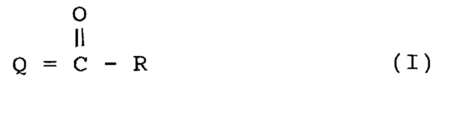 worin bedeuten:
Q den Rest einer aromatischen Verbindung und R eine gerad- oder verzweigtkettige oder cyclische aliphatische Gruppe, eine aromatische Gruppe oder eine araliphatische Gruppe,
durch Umsetzen einer aromatischen Verbindung (mit Ausnahme der Verbindung der Formel: 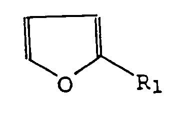 mit R₁ gleich H oder einer kurzkettigen Alkylgruppe), entweder mit
(a) einem gemischten Säureanhydrid der Formel: 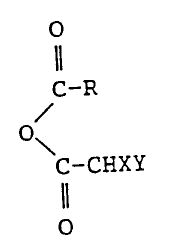 worin bedeuten:
R eine gerad- oder verzweigtkettige oder cyclische aliphatische Gruppe, eine aromatische oder araliphatische Gruppe, wobei (die durch) R (dargestellte Gruppe) einen oder mehrere Substituenten, nämlich ein Halogenatom, eine Alkylgruppe mit 1 bis 10 Kohlenstoffatom(en), eine Alkenylgruppe mit 2 bis 10 Kohlenstoffatomen, eine Alkinylgruppe mit 2 bis 10 Kohlenstoffatomen, eine Alkenyloxylgruppe mit 2 bis 10 Kohlenstoffatomen, eine Alkinyloxylgruppe mit 2 bis 10 Kohlenstoffatomen, eine Alkoxylgruppe mit 1 bis 10 Kohlenstoffatom(en), eine Alkanoyloxylgruppe mit 1 bis 10 Kohlenstoffatom(en), eine Aralkanoyloxylgruppe mit 8 bis 13 Kohlenstoffatomen, eine Alkoxycarbonylgruppe mit 2 bis 11 Kohlenstoffatomen, eine Aralkoxycarbonylgruppe mit 8 bis 13 Kohlenstoffatomen, eine Alkanoylgruppe mit 2 bis 12 Kohlenstoffatomen, eine Aralkanoylgruppe mit 8 bis 13 Kohlenstoffatomen, eine Alkanoylalkoxylgruppe mit 3 bis 13 Kohlenstoffatomen, eine Alkoxyalkoxylgruppe mit 2 bis 12 Kohlenstoffatomen, die Methylendioxygruppe, die Ethylendioxygruppe, eine Alkylthiogruppe mit 1 bis 10 Kohlenstoffatom(en), eine Alkanoylthiogruppe mit 2 bis 10 Kohlenstoffatomen, eine Alkanoylaminogruppe mit 2 bis 10 Kohlenstoffatomen, eine Alkylaminocarbonylgruppe mit 2 bis 10 Kohlenstoffatomen, eine Dialkylaminocarbonylgruppe mit 3 bis 12 Kohlenstoffatomen, eine Aralkylgruppe mit 7 bis 15 Kohlenstoffatomen, eine Aralkyloxylgruppe mit 7 bis 15 Kohlenstoffatomen, eine Dialkylaminogruppe mit 2 bis 8 Kohlenstoffatomen oder eine Phenoxylgruppe enthalten kann;
X ein Wasserstoff-, Chlor- oder Bromatom und
Y ein Chlor- oder Bromatom, oder mit
(b) einer Carbonsäure der Formel:
RCOOH
worin R die zuvor angegebene Bedeutung besitzt, in Gegenwart eines Säureanhydrids der Formel (IV)
(XYCHCO)₂O
worin X und Y die zuvor angegebenen Bedeutungen besitzen,
in Gegenwart eines Bortrifluorid- oder eines Bortrifluoridkomplexkatalysators.

2. Verfahren nach Anspruch 1, wobei die gerad- oder verzweigtkettige oder cyclische aliphatische Gruppe aus einer Alkylgruppe mit 1 bis 18 Kohlenstoffatom(en), einer Alkenylgruppe mit 2 bis 18 Kohlenstoffatomen, einer Alkinylgruppe mit 2 bis 18 Kohlenstoffatomen, einer cyclischen Alkylgruppe mit 3 bis 8 Kohlenstoffatomen, einer cyclischen Alkenylgruppe mit 3 bis 8 Kohlenstoffatomen, einer cyclischen Alkinylgruppe mit 3 bis 8 Kohlenstoffatomen, einer Cycloalkylalkylgruppe mit 3 bis 15 Kohlenstoffatomen, einer Cycloalkylalkenylgruppe mit 3 bis 15 Kohlenstoffatomen, einer Cycloalkylalkinylgruppe mit 3 bis 15 Kohlenstoffatomen, einer Cycloalkenylalkylgruppe mit 3 bis 15 Kohlenstoffatomen, einer Cycloalkinylalkylgruppe mit 3 bis 15 Kohlenstoffatomen oder einer Cycloalkenylalkenylgruppe mit 3 bis 15 Kohlenstoffatomen besteht.

3. Verfahren nach Anspruch 1, wobei die aromatische oder araliphatische Gruppe aus einer Thienylalkylgruppe, einer Thienylalkenylgruppe, einer Aralkylgruppe, einer Thienylalkinylgruppe, einer Furylalkylgruppe, einer Naphthylalkylgruppe, einer Furylalkenylgruppe, einer Aralkenylgruppe, einer Aralkinylgruppe, einer Biphenylylalkylgruppe, einer Biphenylylalkenylgruppe, gegebenenfalls 1-, 2- oder 3-fach substituiert durch Alkylgruppen mit 1 - 12 Kohlenstoffatom(en), Alkenylgruppen mit 1 bis 12 Kohlenstoffatom(en), Alkinylgruppen mit 1 bis 12 Kohlenstoffatom(en), Alkoxylgruppen mit 1 bis 12 Kohlenstoffatom(en), Alkenyloxygruppen mit 1 bis 12 Kohlenstoffatom(en), Alkinyloxylgruppen mit 2 bis 12 Kohlenstoffatomen, die Methylendioxygruppe, die Ethylendioxygruppe, Aralkylgruppen mit 7 bis 12 Kohlenstoffatomen, Aralkyloxylgruppen mit 7 bis 12 Kohlenstoffatomen, die Phenoxylgruppe, Alkyloxycarbonylgruppen mit 2 bis 9 Kohlenstoffatomen, Alkylcarbonyloxylgruppen mit 2 bis 9 Kohlenstoffatomen oder Halogenatome, besteht.

4. Verfahren nach Anspruch 1, wobei die aromatische Gruppe aus einer Phenyl-, Biphenyl-, Naphthyl-, Thienyl- oder Furylgruppe, gegebenenfalls 1-, 2- oder 3-fach substituiert durch Alkylgruppen mit 1 - 12 Kohlenstoffatom(en), Alkenylgruppen mit 1 bis 12 Kohlenstoffatom(en), Alkinylgruppen mit 1 bis 12 Kohlenstoffatom(en), Alkoxylgruppen mit 1 bis 12 Kohlenstoffatom(en), Alkenyloxygruppen mit 1 bis 12 Kohlenstoffatom(en), Alkinyloxylgruppen mit 2 bis 12 Kohlenstoffatomen, die Methylendioxygruppe, die Ethylendioxygruppe, Aralkylgruppen mit 7 bis 12 Kohlenstoffatomen, Aralkyloxylgruppen mit 7 bis 12 Kohlenstoffatomen, die Phenoxylgruppe, Alkyloxycarbonylgruppen mit 2 bis 9 Kohlenstoffatomen, Alkylcarbonyloxylgruppen mit 2 bis 9 Kohlenstoffatomen oder Halogenatome, besteht.

5. Verfahren nach Anspruch 1, wobei es sich bei der aromatischen Verbindung um eine solche handelt, mit der eine Friedel-Crafts-Reaktion durchführbar ist.

6. Verfahren nach Anspruch 5, wobei die aromatische Verbindung ausgewählt ist aus der Gruppe:
(a) Benzol, Biphenyl, Terphenyl, Naphthalin und Anthracen, wobei Benzol, Biphenyl und Terphenyl ein- bis vierfach durch einen mindestens eine elektronenspendende Gruppe enthaltenden Substituenten substituiert sind und Naphthalin und Anthracen unsubstituiert oder ein- oder vierfach durch einen mindestens eine elektronenspendende Gruppe enthaltenden Substituent substituiert sind;
(b) ein Thiophenderivat mit nicht mehr als drei Substituenten;
(c) ein Pyrrol und Pyrrolderivate mit Substituenten am Stickstoffatom und/oder nicht mehr als zwei Substituenten am Kohlenstoffatom;
(d) ein Furanderivat der Formeln: 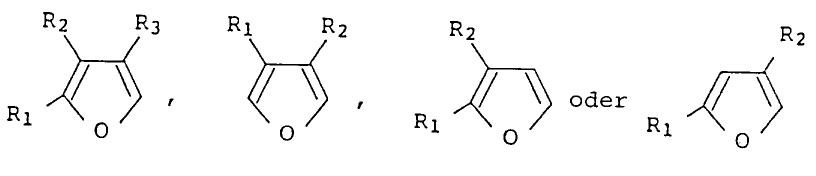 worin R₁, R₂ oder R₃ für einen Substituenten stehen;
(e) eine ein Heteroatom enthaltende aromatische Verbindung der Formeln: 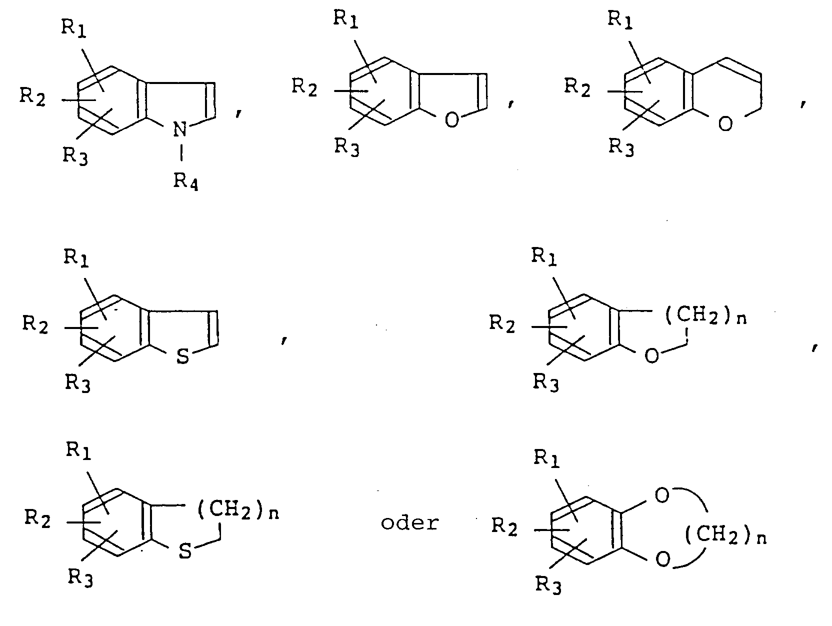 worin R₁, R₂ oder R₃ für einen Substituenten stehen, R₄ ein Wasserstoffatom, eine Niedrigalkyl- oder eine Acylgruppe darstellt und n einer ganzen Zahl von 1 bis 4 entspricht, und
(f) eine einen kondensierten Ring aufweisende aromatische Verbindung der Formeln: 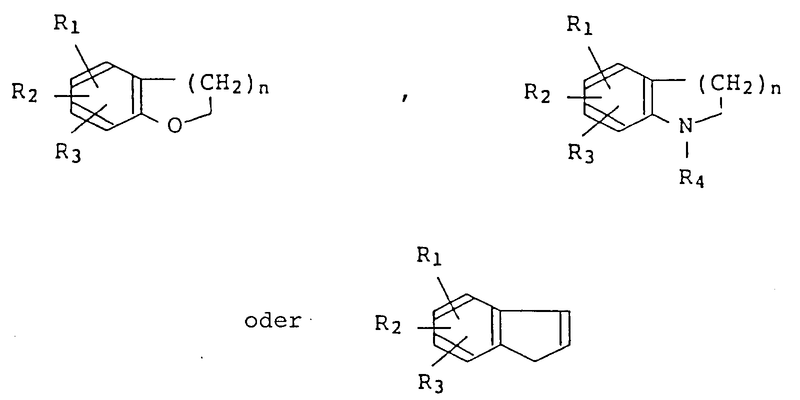 worin R₁, R₂ oder R₃ für einen Substituenten stehen, R₄ eine Niedrigalkylgruppe oder eine Acylgruppe darstellt, wobei gilt, daß mindestens einer der Substituenten aus einer "elektronenspendenden Gruppe" besteht, und n einer ganzen Zahl von 1 bis 4 entspricht.

7. Verfahren nach Anspruch 6, wobei die elektronenspendende Gruppe ausgewählt ist aus der Gruppe:
(a) eine Alkyloxygruppe, Alkenyloxygruppe und Alkinyloxygruppe mit jeweils einer gerad- oder verzweigtkettigen C₁-C₁₂-Alkyl-,-Alkenyl- oder -Alkinylgruppe;
(b) eine gerad- oder verzweigtkettige C₁-C₁₂-Alkanoyloxygruppe;
(c) eine gerad- oder verzweigtkettige C₈-C₁₅-Aralkanoyloxygruppe;
(d) eine substituierte oder unsubstituierte C₇-C₁₈ Aralkyloxygruppe;
(e) eine Alkylthiogruppe und eine Alkylcarbonylthiogruppe mit C₁-C₁₂-Alkyl;
(f) eine Alkylcarbonylalkoxygruppe mit 3 bis 12 Kohlenstoffatomen;
(g) eine Alkylcarbonyloxyalkylgruppe mit 3 bis 12 Kohlenstoffatomen;
(h) eine Alkyloxyalkoxygruppe mit 2 bis 12 Kohlenstoffatomen;
(i) eine Alkyloxycarbonylalkoxygruppe mit 3 bis 12 Kohlenstoffatomen;
(j) eine Alklycarbonyloxyalkoxygruppe mit 3 bis 12 Kohlenstoffatomen;
(k) ein Halogenatom;
(l) Methylendioxy, Ethylendioxy und Phenoxy;
(m) Dialkylamino, Alkylamino, Pyridino, Pyrimidino, Morpholino und n-(Niedrig)alkylpyrrolidino.

8. Verfahren nach Anspruch 6, wobei der Substituent ausgewählt ist aus der Gruppe:
(a) eine Alkyloxygruppe, Alkenyloxygruppe und Alkinyloxygruppe mit jeweils einer gerad- oder verzweigtkettigen C₁-C₁₂-Alkyl-, -Alkenyl- oder -Alkinylgruppe;
(b) eine gerad- oder verzweigtkettige C₁-C₁₂-Alkanoyloxygruppe;
(c) eine gerad- oder verzweigtkettige C₈-C₁₅-Aralkanoyloxygruppe;
(d) eine substituierte oder unsubstituierte C₇-C₁₈-Aralkyloxygruppe;
(e) eine Alkylthiogruppe und Alkylcarbonylthiogruppe mit C₁-C₁₂-Alkyl;
(f) eine Alkylcarbonylalkoxygruppe mit 3 bis 12 Kohlenstoffatomen;
(g) eine Alkylcarbonyloxyalkylgruppe mit 3 bis 12 Kohlenstoffatomen;
(h) eine Alkyloxyalkoxygruppe mit 2 bis 12 Kohlenstoffatomen;
(i) eine Alkyloxycarbonylalkoxygruppe mit 3 bis 12 Kohlenstoffatomen;
(j) eine Alkylcarbonyloxyalkoxygruppe mit 3 bis 12 Kohlenstoffatomen;
(k) ein Halogenatom;
(l) Methylendioxy, Ethylendioxy und Phenoxy;
(m) Dialkylamino, Alkylamino, Pyridino, Pyrimidino, Morpholino und N-(Niedrig)alkylpyrrolidino;
(n) C₁-C₁₂-Alkyl, C₁-C₁₂-Alkenyl und C₁-C₁₂-Alkinyl;
o) C₇-C₁₈-Aralkyl mit/ohne Substituent(en);
(p) eine Alkanoylalkyl- und Alkoxycarbonylalkylgruppe mit 3 bis 12 Kohlenstoffatomen;
(q) C₁-C₁₂-Acylamino;
(r) eine Alkoxyalkylgruppe mit 2 bis 12 und
(s) eine Aralkylcarbonyloxyalkylgruppe mit 9 bis 15 Kohlenstoffatomen.

9. Verfahren nach Anspruch 1, wobei der Bortrifluoridkomplex aus einem Bortrifluorid/Diethylether-Komplex, Bortrifluorid/Essigsäure-Komplex oder Bortrifluorid/Methanol-Komplex besteht.

10. Verfahren nach Anspruch 1, wobei die Menge des Katalysators, bezogen auf das gemischte Säureanhydrid, 0,05 - 1 Äquivalent beträgt.

11. Verfahren nach Anspruch 1, wobei die Menge des Katalysators, bezogen auf die Carbonsäure, 0,02 - 0,2 Äquivalent beträgt.

12. Verfahren nach Anspruch 1, wobei die Menge der aromatischen Verbindung, bezogen auf das gemischte Säureanhydrid, 1,2 - 2 Äquivalente beträgt.

13. Verfahren nach Anspruch 1, wobei die Menge der aromatischen Verbindung, bezogen auf die Carbonsäure, 1,2 - 2 Äquivalente beträgt.

14. Verfahren nach Anspruch 1, wobei die Menge des Säureanhydrids, bezogen auf die Carbonsäure, 1,1 - 1,3 Äquivalente beträgt.

15. Verfahren nach Anspruch 1, wobei das gemischte Säureanhydrid der Formel: 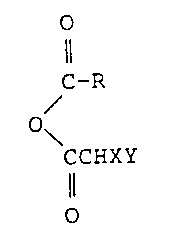 worin R, X und Y die angegebenen Bedeutungen besitzen, durch Umsetzen einer Carbonsäure der Formel:
RCOOH
worin R die zuvor angegebene Bedeutung besitzt, mit einer Halogenessigsäureverbindung der Formel:
XYCHCOZ
worin X für ein Wasserstoff-, Chlor- oder Bromatom steht, Y ein Brom- oder Chloratom darstellt und Z einer Hydroxylgruppe oder einem Chlor- oder Bromatom entspricht, hergestellt wurde.

16. Verfahren nach Anspruch 1 oder 15, wobei es sich bei der Carbonsäure um eine gesättigte gerad- oder verzweigtkettige oder cyclische aliphatische Carbonsäure handelt.

17. Verfahren nach Anspruch 1 oder 15, wobei es sich bei der Carbonsäure um eine ungesättigte gerad- oder verzweigtkettige oder cyclische aliphatische Carbonsäure handelt.

18. Verfahren nach Anspruch 16, wobei es sich bei der aliphatischen Carbonsäure um eine gesättigte alicyclische Carbonsäure mit 4 bis 9 Kohlenstoffatomen handelt.

19. Verfahren nach Anspruch 1 oder 15, wobei die Carbonsäure aus Benzoesäure besteht.

20. Verfahren nach Anspruch 1 oder 15, wobei es sich bei der Carbonsäure um eine ein-, zwei- oder dreifach alkyl- (mit 1 bis 12 Kohlenstoffatom(en)), alkoxyl(mit 1 bis 12 Kohlenstoffatom(en)) oder halogensubstituierte Benzoesäure handelt.

21. Verfahren nach Anspruch 1 oder 15, wobei es sich bei der Carbonsäure um eine Aralkylcarbonsäure handelt.

22. Verfahren nach Anspruch 1 oder 15, wobei es sich bei der Carbonsäure um eine durch eine Alkoxyl- oder Alkoxycarbonylgruppe mit 1 bis 10 Kohlenstoffatom(en) substituierte gerad- oder verzweigtkettige aliphatische Carbonsäure mit 2 bis 19 Kohlenstoffatomen handelt.

23. Verfahren nach Anspruch 15, wobei der Substituent Z der Halogenessigsäureverbindung aus einem Chlor- oder Bromatom besteht.

24. Verfahren nach Anspruch 23, wobei die Umsetzung in Gegenwart einer organischen Base erfolgt.

25. Verfahren nach Anspruch 24, wobei die organische Base aus Triethylamin, Pyridin oder Diethylanilin besteht.

26. Verfahren nach Anspruch 23, wobei die Menge der Halogenessigsäureverbindung, bezogen auf die Carbonsäure, 1 - 1,5 Äquivalent(e) beträgt.

27. Verfahren nach Anspruch 24, wobei die Menge der organischen Base, bezogen auf die Carbonsäure, 1 - 1,5 Äquivalent(e) beträgt.

28. Verfahren nach Anspruch 23, wobei die Reaktionstemperatur im Bereich von -20 bis 50°C liegt.

## Revendications

1. Procédé de préparation d'un composé acylaromatique représenté par la formule 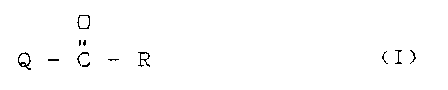 dans laquelle Q est un résidu de composé aromatique et R est un groupement aliphatique en chaîne droite, ramifié ou cyclique, un groupement aromatique ou un groupement araliphatique,
consistant à faire réagir, en présence de trifluorure de bore ou d'un catalyseur constitué par un complexe de trifluorure de bore, un composé aromatique, à l'exception du composé représenté par la formule 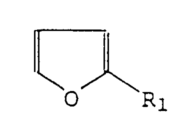 dans laquelle R₁ = H ou groupement alkyle inférieur,
soit avec
a) un anhydride d'acide mixte représenté par la formule 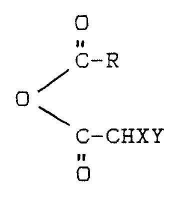 dans laquelle R est un groupement aliphatique en chaîne droite, ramifié ou cyclique, un groupement aromatique ou araliphatique, R pouvant porter un ou plusieurs substituants tels que des atomes d'halogène, des groupements alkyle à 1-10 atomes de carbone, des groupements alcényle à 2-10 atomes de carbone, des groupements alcynyle à 2-10 atomes de carbone, des groupements alcényloxy à 2-10 atomes de carbone, des groupements alcynyloxy à 2-10 atomes de carbone, des groupements alcoxy à 1-10 atomes de carbone, des groupements alcanoyloxy à 1-10 atomes de carbone, des groupements aralcanoyloxy à 8-13 atomes de carbone, des groupements alcoxycarbonyle à 2-11 atomes de carbone, des groupements aralcoxycarbonyle à 8-13 atomes de carbone, des groupements alcanoyle à 2-12 atomes de carbone, des groupements aralcanoyle à 8-13 atomes de carbone, des groupements alcanoylalcoxy à 3-13 atomes de carbone, des groupements alcoxyalcoxy à 2-12 atomes de carbone, des groupements méthylènedioxy, des groupements éthylènedioxy des groupements alkylthio à 1-10 atomes de carbone, des groupements alcanoylthio à 2-10 atomes de carbone, des groupements alcanoylamino à 2-10 atomes de carbone, des groupements alkylaminocarbonyle à 2-10 atomes de carbone, des groupements dialkylaminocarbonyle à 3-12 atomes de carbone, des groupements aralkyle à 7-15 atomes de carbone, des groupements aralkyloxy à 7-15 atomes de carbone, des groupements dialkylamino à 2-8 atomes de carbone ou des groupements phénoxy; X est un atome d'hydrogène, de chlore ou de brome; et Y est un atome de chlore ou de brome;
soit avec
b) un acide carboxylique représenté par la formule (III)
RCOOH (III)
dans laquelle R a la même signification que ci-dessus, en présence d'un anhydride d'acide représenté par la formule (IV)
(XYCHCO)₂O (IV)
dans laquelle X et Y ont les mêmes significations que précédemment.

2. Procédé selon la revendication 1, dans lequel le groupement aliphatique en chaîne droite, ramifié ou cyclique est un groupement alkyle à 1-18 atomes de carbone, un groupement alcényle à 2-18 atomes de carbone, un groupement alcynyle à 2-18 atomes de carbone, un groupement alkyle cyclique à 3-8 atomes de carbone, un groupement alcényle cyclique à 3-8 atomes de carbone, un groupement alcynyle cyclique à 3-8 atomes de carbone, un groupement cycloalkylalkyle à 3-15 atomes de carbone, un groupement cycloalkylalcényle à 3-15 atomes de carbone, un groupement cycloalkylalcynyle à 3-15 atomes de carbone, un groupement cycloalcénylalkyle à 3-15 atomes de carbone, un groupement cycloalcynylalkyle à 3-15 atomes de carbone ou un groupement cycloalcénylalcényle à 3-15 atomes de carbone.

3. Procédé selon la revendication 1, dans lequel le groupement aromatique ou araliphatique est un groupement thiénylalkyle, un groupement thiénylalcényle, un groupement aralkyle, un groupement thiénylalcynyle, un groupement furylalkyle, un groupement naphtylalkyle, un groupement furylalcényle, un groupement aralcényle, un groupement aralcynyle, un groupement biphénylylalkyle, un groupement biphénylylalcényle non substitué ou substitué une fois, deux fois ou trois fois par des groupements alkyle à 1-12 atomes de carbone, des groupements alcényle à 1-12 atomes de carbone, des groupements alcynyle à 1-12 atomes de carbone, des groupements alcoxy à 1-12 atomes de carbone, des groupements alcényloxy à 1-12 atomes de carbone, des groupements alcynyloxy à 2-12 atomes de carbone, un groupement méthylènedioxy, un groupement éthylènedioxy, des groupements aralkyle à 7-12 atomes de carbone, des groupements aralkyloxy à 7-12 atomes de carbone, un groupement phénoxy, des groupements alkyloxycarbonyle à 2-9 atomes de carbone, des groupements alkylcarbonyloxy a 2-9 atomes de carbone et des atomes d'halogène.

4. Procédé selon la revendication 1, dans lequel le groupement aromatique est un groupement phényle, biphényle, naphtyle, thiényle ou furyle non substitué ou substitué une fois, deux fois ou trois fois par des groupements alkyle à 1-12 atomes de carbone, des groupements alcényle à 1-12 atomes de carbone, des groupements alcynyle à 1-12 atomes de carbone, des groupements alcoxy à 1-12 atomes de carbone, des groupements alcényloxy à 1-12 atomes de carbone, des groupements alcynyloxy à 2-12 atomes de carbone, un groupement méthylènedioxy, un groupement éthylènedioxy, des groupements aralkyle à 7-12 atomes de carbone, des groupements aralkyloxy à 7-12 atomes de carbone, un groupement phénoxy, des groupements alkyloxycarbonyle à 2-9 atomes de carbone, des groupements alkylcarbonyloxy à 2-9 atomes de carbone et des atomes d'halogène.

5. Procédé selon la revendication 1, dans lequel le composé aromatique est un composé auquel le réaction de Friedel-Crafts est applicable.

6. Procédé selon la revendication 5, dans lequel le composé aromatique est choisi dans le groupe constitué par
a) le benzène, le biphényle, le terphényle, le naphtalène et l'anthracène, parmi lesquels le benzène, le biphényle et le terphényle sont substitués une à quatre fois par un substituant contenant au moins un groupement donneur d'électrons, et le naphtalène et l'anthracène sont non substitués ou substitués une à quatre fois par des substituants contenant au moins un groupement donneur d'électrons,
b) un dérivé du thiophène ne portant pas plus de trois substituants,
c) du pyrrole et un dérivé du pyrrole portant un substituant sur l'atome d'azote et/ou portant des substituants en un nombre ne dépassant pas 2 sur l'atome de carbone,
d) un dérivé du furanne représenté par la formule 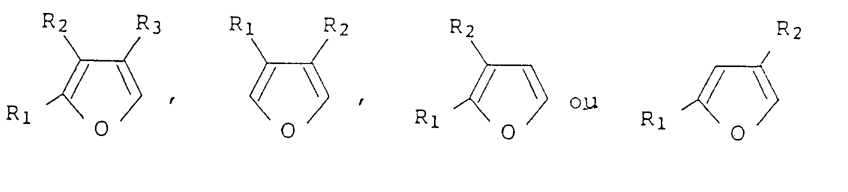 dans laquelle R₁, R₂ ou R₃ représente un substituant,
e) un composé aromatique contenant un hétéroatome, représenté par la formule 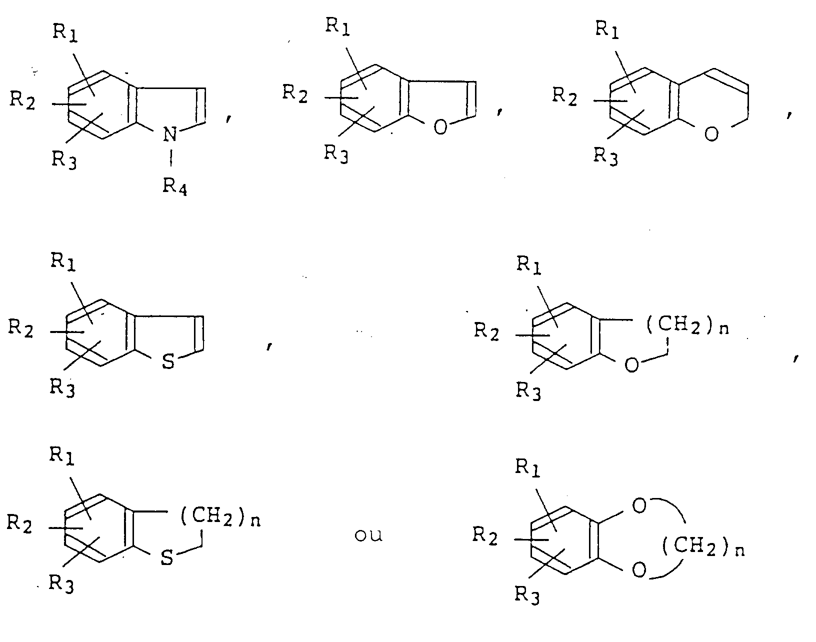 dans laquelle R₁, R₂ ou R₃ représente un substituant, R₄ représente un atome d'hydrogène, un groupement alkyle inférieur ou acyle et n est un nombre entier de 1 à 4, et
f) un composé aromatique à noyaux condensés représenté par la formule 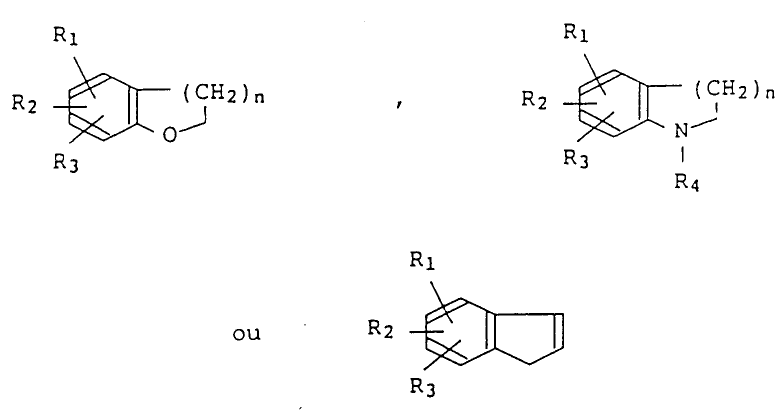 dans laquelle R₁, R₂ ou R₃ représente un substituant et R₄ représente un groupement alkyle inférieur ou un groupement acyle, étant spécifié que l'un au moins des substituants est un "groupement donneur d'électrons", et n est un nombre entier de 1 à 4.

7. Procédé selon la revendication 6, dans lequel le groupement donneur d'électrons est choisi dans le groupe constitué par
a) un groupement alkyloxy, un groupement alcényloxy et un groupement alcynyloxy comportant respectivement un groupement alkyle, alcényle ou alcynyle en C₁-C₁₂ en chaîne droite ou ramifié,
b) un groupement alcanoyloxy en C₁-C₁₂ en chaîne droite ou ramifié,
c) un groupement aralcanoyloxy en C₈-C₁₅ en chaîne droie ou ramifié,
d) un groupement aralkyloxy en C₇-C₁₈ substitué ou non substitué,
e) un groupement alkylthio et un groupement alkylcarbonylthio renfermant un reste alkyle en C₁-C₁₂,
f) un groupement alkylcarbonylalcoxy à 3-12 atomes de carbone,
g) un groupement alkylcarbonyloxyalkyle à 3-12 atomes de carbone,
h) un groupement alkyloxyalcoxy à 2-12 atomes de carbone,
i) un groupement alkyloxycarbonylalcoxy à 3-12 atomes de carbone,
j) un groupement alkylcarbonyloxyalcoxy à 3-12 atomes de carbone,
k) un atome d'halogène,
l) un groupement méthylènedioxy, éthylènedioxy ou phénoxy,
m) un groupement dialkylamino, alkylamino, pyridino, pyrimidino, morpholino ou N-(alkyl inférieur)-pyrrolidino.

8. Procédé selon la revendication 6, dans lequel le substituant est choisi dans le groupe constitué par
a) un groupement alkyloxy, un groupement alcényloxy et un groupement alcynyloxy renfermant respectivement un groupement alkyle, alcényle ou alcynyle en C₁-C₁₂ en chaîne droite ou ramifié,
b) un groupement alcanoyloxy en C₁-C₁₂ en chaîne droite ou ramifié,
c) un groupement aralcanoyloxy en C₈-C₁₅ en chaîne droite ou ramifié,
d) un groupement aralkyloxy en C₇-C₁₈ substitué ou non substitué,
e) un groupement alkylthio et un groupement alkylcarbonylthio renfermant un reste alkyle en C₁-C₁₂,
f) un groupement alkylcarbonylalcoxy à 3-12 atomes de carbone,
g) un groupement alkylcarbonyloxyalkyle à 3-12 atomes de carbone,
h) un groupement alkyloxyalcoxy à 2-12 atomes de carbone,
i) un groupement alkyloxycarbonylalcoxy à 3-12 atomes de carbone,
j) un groupement alkylcarbonyloxyalcoxy à 3-12 atomes de carbone,
k) un atome d'halogéne,
l) un groupement méthylènedioxy, éthylènedioxy ou phénoxy,
m) un groupement dialkylamino, alkylamino, pyridino, pyrimidino, morpholino ou N-(alkyl inférieur)-pyrrolidino,
n) un groupement alkyle en C₁-C₁₂, alcényle en C₁-C₁₂ ou alcynyle en C₁-C₁₂,
o) un groupement aralkyle en C₇-C₁₈ avec/sans substituant,
p) un groupement alcanoylalkyle ou alcoxycarbonylalkyle à 3-12 atomes de carbone,
q) un groupement acylamino en C₁-C₁₂,
r) un groupement alcoxyalkyle à 2-12 atomes de carbone et
s) un groupement aralkylcarbonyloxyalkyle à 9-12 atomes de carbone.

9. Procédé selon la revendication 1, dans lequel le complexe de trifluorure de bore est un complexe oxyde de diéthyle-trifluorure de bore, un complexe acide acétique-trifluorure de bore ou un complexe méthanoltrifluorure de bore.

10. Procédé selon la revendication 1, dans lequel la quantité dudit catalyseur est comprise entre 0,05 et 1 équivalent par rapport audit anhydride d'acide mixte.

11. Procédé selon la revendication 1, dans lequel la quantité dudit catalyseur est comprise entre 0,02 et 0,2 équivalent par rapport audit acide carboxylique.

12. Procédé selon la revendication 1, dans lequel la quantité dudit composé aromatique est comprise entre 1,2 et 2 équivalents par rapport audit anhydride d'acide mixte.

13. Procédé selon la revendication 1, dans lequel la quantité dudit composé aromatique est comprise entre 1,2 et 2 équivalents par rapport audit acide carboxylique.

14. Procédé selon la revendication 1, dans lequel la quantité dudit anhydride d'acide est comprise entre 1,1 et 1,3 équivalent par rapport audit acide carboxylique.

15. Procédé selon la revendication 1, dans lequel ledit anhydride d'acide mixte représenté par la formule 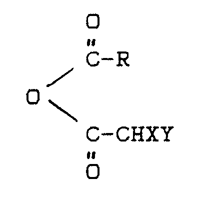 dans laquelle R, X et Y sont tels que définis précédemment, est préparé par réaction d'un acide carboxylique représenté par la formule
RCOOH
dans laquelle R est tel que défini précédemment, avec un composé acide halogénacétique représenté par la formule
XYCHCOZ
dans laquelle X est un atome d'hydrogène, de chlore ou de brome, Y est un atome de brome ou de chlore et Z est un groupement hydroxy ou un atome de chlore ou de brome.

16. Procédé selon la revendication 1 ou 15, dans lequel ledit acide carboxylique est un acide carboxylique aliphatique en chaîne droite, ramifié ou cyclique.

17. Procédé selon la revendication 1 ou 15, dans lequel ledit acide carboxylique est un acide carboxylique aliphatique insaturé en chaîne droite, ramifié ou cyclique.

18. Procédé selon la revendication 16, dans lequel ledit acide carboxylique aliphatique est un acide carboxylique alicyclique saturé à 4-9 atomes de carbone.

19. Procédé selon la revendication 1 ou 15, dans lequel ledit acide carboxylique est l'acide benzoïque.

20. Procédé selon la revendication 1 ou 15, das lequel ledit acide carboxylique est un acide benzoïque substitué par un, deux ou trois groupements alkyle à 1-12 atomes de carbone, groupements alcoxy à 1-12 atomes de carbone ou atomes d'halogène.

21. Procédé selon la revendication 1 ou 15, dans lequel ledit acide carboxylique est un acide carboxylique aralkylique.

22. Procédé selon la revendication 1 ou 15, dans lequel ledit acide carboxylique est un acide carboxylique aliphatique en chaîne droite ou ramifié à 2-19 atomes de carbone, substitué par un groupement alcoxy ou un groupement alcoxycarbonyle à 1-10 atomes de carbone.

23. Procédé selon la revendication 15, dans lequel le substituant Z dudit composé acide halogénacétique est un atome de chlore ou de brome.

24. Procédé selon la revendication 23, dans lequel ladite réaction est menée en présence d'une base organique.

25. Procédé selon la revendication 24, dans lequel ladite base organique est la triéthylamine, la pyridine ou la diéthylaniline.

26. Procédé selon la revendication 23, dans lequel la quantité dudit composé acide halogénacétique est comprise entre 1 et 1,5 équivalent par rapport audit acide carboxylique.

27. Procédé selon la revendication 24, dans lequel la quantité de ladite base organique est comprise entre 1 et 1,5 équivalent par rapport audit acide carboxylique.

28. Procédé selon la revendication 23, dans lequel la température de réaction est comprise entre -20 et 50°C.
